# EUROPÄISCHE PATENTSCHRIFT

(11) **EP 4 243 882 B1**
(45) Veröffentlichungstag und Bekanntmachung des Hinweises auf die Patenterteilung: **01.10.2025**
(21) Anmeldenummer: 21805997.0
(22) Anmeldetag: 16.11.2021
(51) Int. Cl.: A61L 2/07, A61L 2/08, B01D 65/02, A61L 2/26

(54) **VERFAHREN ZUR SICHERSTELLUNG EINER MIKROBIOLOGISCHEN REINHEIT EINER EINWEG-VORRICHTUNG**
METHOD FOR ENSURING A MICROBIOLOGICAL PURITY OF A DISPOSABLE DEVICE
PROCÉDÉ DE MAINTIEN DE LA PURETÉ MICROBIOLOGIQUE D'UN DISPOSITIF JETABLE

(30) Priorität: 16.11.2020 EP 20207785
(43) Veröffentlichungstag der Anmeldung: 20.09.2023
(73) Patentinhaber: Sartorius Stedim Biotech GmbH, 37079 Göttingen (DE)
(72) Erfinder: WORTMEYER, Johannes, 37079 Göttingen (DE); LOEWE, Thomas, 37079 Göttingen (DE); MIETH, Florian, 37079 Göttingen (DE)
(74) Vertreter: Novagraaf International SA
(86) Internationale Anmeldenummer: PCT/EP2021/081835
(87) Internationale Veröffentlichungsnummer: WO 2022/101499

(56) Entgegenhaltungen:
- WO-A1-2016/169630
- DE-A1- 102015 114 004
- DE-U1- 29 908 080
- US-A1- 2017 224 432
- MDI: "AseptiCap", 1 February 2017 (2017-02-01), XP055832762, Retrieved from the Internet <URL:https://www.gesfilter.com/wp-content/uploads/2017/02/0.2-AseptiCap-KL-KS-Datasheet.pdf> [retrieved on 20210818]

## Beschreibung

Die Erfindung betrifft ein Verfahren zur Sicherstellung einer mikrobiologischen Reinheit einer Einweg-Vorrichtung zur Durchführung eines bioverfahrenstechnischen Prozesses, insbesondere einer Einweg-Filtrationsvorrichtung.

Die vorliegende Erfindung richtet sich auf groß dimensionierte, komplexe Vorrichtungen für die kommerzielle Fertigung (Produktionsmaßstab). Solche Vorrichtungen sind insbesondere abzugrenzen von kleineren, leichter handhabbaren Vorrichtungen, die für experimentelle Zwecke bzw. zur Entwicklung neuer Prozesse verwendet werden (Labormaßstab).

Eine Einweg-Vorrichtung im Sinne der Erfindung ist eine zum einmaligen Gebrauch vorgesehene Vorrichtung, die anschließend nicht für eine erneute Verwendung gereinigt und sterilisiert, sondern in der Regel vollständig entsorgt wird. Dementsprechend unterscheidet sich eine Einweg-Vorrichtung hinsichtlich der für ihre einzelnen Komponenten verwendeten Materialien (vorwiegend Kunststoffe) von einer wiederverwendbaren Vorrichtung, die z. B. Komponenten aus Edelstahl aufweist.

Unter einer Separationseinheit ist im Zusammenhang der vorliegenden Erfindung z. B. eine Filtercapsule, eine Chromatographiesäule oder ein Membranadsorber zu verstehen. Grundsätzlich ist das Verfahren aber auch bei Einweg-Vorrichtungen mit anderen Funktionseinheiten anwendbar (fällt jedoch nicht unter die Ansprüche), insbesondere wenn diese - im Gegensatz zu anderen Komponenten der Einweg-Vorrichtung - nicht für eine Sterilisierung durch Gammastrahlung geeignet sind. Hierauf wird später noch eingegangen.

Grundsätzlich besteht der Wunsch eines Anwenders einer Einweg-Vorrichtung zur Durchführung eines bioverfahrenstechnischen Prozesses, insbesondere einer Einweg-Filtrationsvorrichtung, dass die Einweg-Vorrichtung nach der Auslieferung sofort einsatzbereit ist. Voraussetzung hierfür ist in der Regel, dass die Einweg-Vorrichtung bestimmten Anforderungen an die mikrobiologische Reinheit genügt. In vielen Anwendungsfällen ist diese unverzichtbar, denn ansonsten besteht die Gefahr, dass die Funktions- bzw. Leistungsfähigkeit der Separationseinheiten nicht gewährleistet und/oder dass das Zielprodukt verunreinigt ist. Letzteres ist besonders prekär, wenn die Verunreinigung dem Zielprodukt ähnlich ist und somit auch nachträglich nicht effektiv herausgefiltert werden kann.

Für den Anwender bedeutet es jedoch eine zusätzliche Belastung, wenn er selbst für die gewünschte mikrobiologische Reinheit der Einweg-Vorrichtung sorgen muss. Es besteht daher der Wunsch, dass die Einweg-Vorrichtung bereits auf Seiten des Herstellers sterilisiert wird. Damit die Einweg-Vorrichtung in sterilem Zustand an den Anwender ausgeliefert werden kann, muss sie mit einer Sterilbarriere versehen werden, die gleichzeitig als Verpackung für den Transport dienen kann.

Die Sterilisierung einer gesamten Einweg-Vorrichtung kann jedoch aus verschiedenen Gründen problematisch sein. Wenn eine Einweg-Vorrichtung Separationseinheiten oder andere Funktionseinheiten enthält, die nicht auf die gleiche Weise sterilisiert werden können wie die anderen Medium-berührenden Komponenten der Einweg-Vorrichtung, sind getrennte Sterilisierungsvorgänge für die jeweiligen Komponenten erforderlich. Das bedeutet, dass die Einweg-Vorrichtung nicht nach dem Zusammenbau als Ganzes sterilisiert werden kann.

Grundsätzlich ist eine Sterilisierung durch Gammastrahlung vorteilhaft, da sie die Abmessungen der Komponenten nicht beeinträchtigt (Maßhaltigkeit). Gewisse Materialien, die bei Separationseinheiten z. B. als Filter- oder Membranmaterial verwendet werden, wie etwa Polytetrafluorethylen (PTFE), Polypropylen (PP) oder Polyvinylchlorid (PVC), sind jedoch für eine Sterilisierung durch Gammastrahlung nicht geeignet, da sie infolge der Bestrahlung wichtige Materialeigenschaften verlieren, insbesondere ihre mechanische Stabilität. Dies ist z.B. von Bedeutung bei Komponenten, die einer starken oder schlagartigen mechanischen Belastung unterliegen, wie etwa Konnektoren an Schlauchenden, die auf den Boden fallen können. Ebenso darf es bei fluidführenden Komponenten infolge einer Gammabestrahlung nicht zu einer kritischen Verunreinigung durch "extractables" (chemische Verbindungen, die unter extremen Bedingungen aus dem Material der Komponente extrahiert werden) kommen. Ebenfalls nicht geeignet für eine Gammasterilisierung sind optische Bauteile, z.B. Schaugläser oder Ports aus transparenten oder transluzenten Kunststoffen für optische Sensoren oder für eine visuelle Kontrolle, die sich bei einer Bestrahlung verfärben.

Separationseinheiten (und andere Komponenten), die aufgrund ihrer Materialien oder den weiteren oben genannten Gründen nicht für eine Gammasterilisierung infrage kommen, müssen deshalb auf andere Weise sterilisiert werden, vorzugsweise durch Heißdampf in einem Autoklav. Die Heißdampfsterilisierung hat jedoch den Nachteil, dass der Überdruck im Autoklav zu einer unerwünschten plastischen Verformung der Separationseinheiten führen kann. So sind etwa Kunststoffklammern und -klemmen, die die eine Pressverbindung erzeugen, in der Regel nicht für das Autoklavieren geeignet. Bei hohen Temperaturen kriechen die Kunststoffe stark, so dass die Dichtheit der Pressverbindung nicht mehr garantiert werden kann. Ein Nachziehen der Klammer oder Klemme ist meist nur bedingt oder gar nicht möglich.

Nicht autoklavierbar, aber gammasterilisierbar sind nicht-hitzebeständige Materialien wie Polyethylen (PE), aus denen z.B. Konnektoren oder Behälter gefertigt sind. Ebenfalls nicht autoklavierbar sind verschraubte oder verklebte Komponenten, wenn die Wärmeausdehnungen stark unterschiedlich sind. Nur bedingt autoklavierbar sind z.B. Schläuche aus thermoplastischen Elastomeren, da sie nach dem Autoklavieren in ihrer Biegung, in der sie im Autoklav lagen, "eingefroren" bleiben.

In jedem Fall kann ein Fachmann anhand der Materialien, aus denen eine für einen bioverfahrenstechnischen Prozess vorgesehene Komponente gebildet ist, und anhand der vorgesehenen Verwendung der Komponente beurteilen, ob diese für eine Gammasterilisierung oder ein Autoklavieren geeignet ist oder nicht.

Wenn die Einweg-Vorrichtung als Ganzes ausgeliefert werden soll, besteht außerdem das Problem, dass vor, während und nach dem Zusammenbau der in getrennten Prozessen sterilisierten Separationseinheiten und übrigen Komponenten Verunreinigungen in das Innere der Separationseinheiten und der anderen Komponenten gelangen können.

DE 299 08 080 U1 betrifft eine einzeln verpackte sterile Einweg-Filterkapsule zur Sterilfiltration, die über einen sterilisierten Schlauch und eine sterilisierte Schnellkupplung an einen sterilen Einweg-Container (Beutel) angeschlossen wird. Ausgehend davon, dass der Anwender bisher sämtliche Einzelteile einer Vorrichtung zur Filtration mit einem Sterilfilter, also neben der Filterkapsule auch den Schlauch, die Schnellkupplung etc. selbst sterilisieren oder steril erwerben und dann beim Zusammenbau sicherstellen muss, dass die Gesamtkonfiguration auch steril ist, wird vorgeschlagen, dass wenigstens die Filterkapsule zusammen mit installiertem Schlauch und der Schnellkupplung als komplette Einheit verpackt und sterilisiert wird. Dabei kommt jedoch nur ein bestimmtes Sterilisationsverfahren zum Einsatz. Das bedeutet, dass die Materialien aller Komponenten der Vorrichtung durch die eine einzelne Sterilisationsmethode sterilisierbar sein müssen, ohne Schaden zu nehmen.

DE 10 2015 114004 A1 stellt eine Einweg-Filtrationsvorrichtung mit einer Mehrzahl von Einweg-Filterkapsulen vor, die durch Leitungen miteinander verbunden sind und von einer starren Halterung getragen werden. Damit die vormontierte Filtrationsvorrichtung ohne großen Aufwand sterilisiert werden kann, wird vorgeschlagen, wenigstens die Halterung und die Leitungen zwischen den Filterkapsulen aus einem sterilisierbaren Material zu bilden, damit die Halterung mit den darin montierten Filterkapsulen und Leitungen in einem Schritt sterilisiert werden können. Entsprechend den bewährten Sterilisationsverfahren sollte das Material vorzugsweise durch Gammastrahlen oder Heißdampf sterilisierbar sein, wobei die Ausbildung aller vorgenannten Komponenten möglichst aus demselben Material besonders vorteilhaft ist. Die Verwendung einer sterilisierbaren Verpackung erlaubt es, die Vorrichtung samt Verpackung im sterilisierten Zustand zu lagern und zu transportieren und beim Anwender schnell auszupacken, aufzustellen und in Betrieb zu nehmen.

WO 2016/169630 A1 beschäftigt sich mit dem Problem, Chromatographiematerial zu sterilisieren. Die bekannten Sterilisationsverfahren basierend z. B. auf Heißdampf oder Gammastrahlung weisen die bekannten Schwierigkeiten hinsichtlich Veränderung der Materialeigenschaften auf und insbesondere die mikroporösen Polymermembranen, welche als Chromatographiematerial zum Einsatz kommen, sind für Schädigung, Verfärbungen oder Funktionseinschränkung/-verlust durch die Sterilisation sehr anfällig. Auch bekannte chemische Verfahren zur Sterilisation durch Spülen mit Lauge (Sanitisierung) sind nur begrenzt einsetzbar. Es wird deshalb eine neue Behandlungsart des Chromatographiematerials vorgeschlagen, welche eine breitere Anwendbarkeit der Sterilisation basierend auf Gammabestrahlung ermöglicht. Das Chromatographiematerial wird hierfür vor Anwendung der Bestrahlung mit einer wässrigen, basisch gepufferten Salzlösung behandelt und optional getrocknet. Diese Vorbehandlung stellt eine Art Konservierung dar. Änderungen der Materialeigenschaften durch die Gammastrahlung sollen dadurch verringert oder sogar vermieden werden.

US 2017/224432 A1 offenbart eine Filtermodulverpackungseinheit, bei der ein Hart-Weich-Blister für eine sterile Verpackung eines Filtermoduls/Dialysators verwendet wird. Eine Primärverpackung schließt sich eng an das verpackte Filtermodul/den Dialysator an. Ein korrespondierender, formähnlicher Tray passt zur und unterstützt das Filtermodul/den Dialysator im Karton, so dass konstruktiv empfindliche Stellen des Filtermoduls/Dialysators (Protection Caps und Konnektoren) ohne Berührung zur Verpackung und/oder einer benachbarten Verpackungseinheit liegen.

Das Mdi Data Sheet "AseptiCap KL/KS" (https://www.qesfilter.com/wpcontent/uploads/2017/02/0.2-AseptiCap-KL-KS-Datasheet.pdf) betrifft Filterkapsulen, bei denen die Integrität der Verpackung während des Transports dadurch gewährleistet werden soll, dass die mit Entlüftungskappen versehenen Filterkapsulen in Polyethylenbeuteln verpackt sind. Durch diese Verpackung soll auch eine Verunreinigung durch Partikel bei der Verbringung in Reinraum- oder Prozessbereiche verhindert werden.

Vor diesem Hintergrund ist es die Aufgabe der Erfindung, die Sicherstellung einer mikrobiologischen Reinheit einer Einweg-Vorrichtung zur Durchführung eines bioverfahrenstechnischen Prozesses, insbesondere einer Einweg-Filtrationsvorrichtung, vor deren Inbetriebnahme zu verbessern und zu vereinfachen.

Gelöst wird diese Aufgabe durch ein Verfahren mit den Merkmalen des Anspruchs 1. Vorteilhafte und zweckmäßige Ausgestaltungen des erfindungsgemäßen Verfahrens sind in den zugehörigen Unteransprüchen angegeben.

Das erfindungsgemäße Verfahren zur Sicherstellung einer mikrobiologischen Reinheit ist für eine große Einweg-Vorrichtung zur Durchführung eines bioverfahrenstechnischen Prozesses in der kommerziellen Fertigung mit einem großen Mediumdurchsatz vorgesehen, wobei die Einweg-Vorrichtung wenigstens eine gammasterilisierbare Komponente, die aus für eine Sterilisierung durch Gammastrahlung geeigneten Materialien gebildet ist, und mehrere Separationseinheiten in Form von Filtercapsulen, Chromatographiesäulen oder Membranadsorbern, von denen wenigstens eine ein für eine Sterilisierung durch Gammastrahlung ungeeignetes Material enthält und somit nicht-gammasterilisierbar ist. Sowohl die Komponente als auch die Separationseinheiten weisen jeweils einen Bereich auf, der bei der Durchführung des bioverfahrenstechnischen Prozesses mit einem Prozess-Medium in Berührung kommt.

Das erfindungsgemäße Verfahren umfasst folgende Schritte: a) Sterilisieren der gammasterilisierbaren Komponente und der gammasterilisierbaren Separationseinheit(en) durch Gammastrahlung; b) Schützen des Medium-berührenden Bereichs der gammasterilisierbaren Komponente und der gammasterilisierbaren Separationseinheit(en) jeweils durch eine Sterilbarriere; c) Sterilisieren der nicht-gammasterilisierbaren Separationseinheit(en) mit Heißdampf einzeln oder in einem Verbund; d) Schützen des Medium-berührenden Bereichs der nicht-gammasterilisierbaren Separationseinheit(en) jeweils durch eine Sterilbarriere; e) Entfernen der Sterilbarrieren; und f) Montieren der gammasterilisierten Komponente und der gammasterilisierten Separationseinheit(en) sowie der Heißdampf-sterilisierten Separationseinheit(en) in der Einweg-Vorrichtung unmittelbar nach dem Entfernen der Sterilbarrieren. Die wenigstens eine gammasterilisierbare Komponente umfasst ein starres Verbindungsrohr mit Fluidanschlüssen, an denen nach Zusammenbau der Einweg-Vorrichtung mehrere Separationseinheiten angeschlossen sind.

Die Erfindung beruht auf der Erkenntnis, dass bei einer Einweg-Vorrichtung mit einer oder mehreren Separationseinheiten, die nicht nach dem Zusammenbau als Ganzes sterilisiert werden kann, die Gefahr einer Verunreinigung in der Phase vor und während des Zusammenbaus der Einweg-Vorrichtung am größten ist. Die Erfindung sieht deshalb besondere Schutzmaßnahmen vor:

Die gammasterilisierbare Komponente und die nicht-gammasterilisierbare(n) Separationseinheit(en) werden in getrennten Vorgängen sterilisiert, und die Medium-berührenden Bereiche der gammasterilisierbaren Komponente und der nicht-gammasterilisierbaren Separationseinheit(en) werden jeweils durch eine Sterilbarriere geschützt. Eine solche Sterilbarriere kann insbesondere eine Hülle oder eine Verpackung für die gesamte Komponente bzw. Separationseinheit(en) sein, oder ein Verschluss, wie etwa eine Blindkappe, ein Sterilkonnektor oder eine Umhüllung, der/die den jeweiligen Medium-berührenden Bereich schützt, wie später noch genauer erläutert wird.

Erst unmittelbar vor dem Montieren der gammasterilisierten Komponente und der Heißdampf-sterilisierten Separationseinheit(en) werden die Sterilbarrieren entfernt.

Sofern nach dem Entfernen der Sterilbarrieren keine weiteren Schutzmaßnahmen vorgesehen sind, wie etwa zusätzliche Verschlüsse, auf die später noch eingegangen wird, sollte der Zeitraum zwischen dem Entfernen einer Sterilbarriere und dem Montieren der zugehörigen gammasterilisierten Komponente bzw. der gammasterilisierten Separationseinheit(en) bzw. der zugehörigen Heißdampf-sterilisierten Separationseinheit(en) je nach Partikelkonzentration in der Umgebung kürzer als vier Minuten, vorzugsweise kürzer als zwei Minuten, weiter vorzugsweise kürzer als eine Minute und noch weiter vorzugsweise kürzer als eine halbe Minute sein. Praxistests haben ergeben, dass bei Einhaltung dieser Zeiträume die Gefahr einer Verunreinigung der Medium-berührenden Bereiche der Einweg-Vorrichtung sehr effektiv gesenkt wird.

Grundsätzlich können die wenigstens eine nicht-gammasterilisierbare Separationseinheit und die wenigstens eine gammasterilisierbare Komponente zu unterschiedlichen Zeiten sterilisiert werden, ohne dass eine Reihenfolge zu beachten ist.

Gemäß einem Aspekt der Erfindung kommt zusätzlich zu den Sterilbarrieren (unter Umständen kann auch eine gemeinsame Sterilbarriere sowohl für die wenigstens eine gammasterilisierbare Komponente als auch für die wenigstens eine nicht-gammasterilisierbare Separationseinheit vorgesehen sein) wenigstens ein Verschluss zum Einsatz, der den Medium-berührenden Bereich der gammasterilisierbaren Komponente bzw. der nicht-gammasterilisierbaren Separationseinheit(en) abdeckt. Ein solcher, von der Sterilbarriere unabhängiger Verschluss muss keine Sterilbarriere bilden, solange er zumindest dazu beiträgt, dass die Wahrscheinlichkeit eines unerwünschten Partikeleintrags signifikant gesenkt wird. Nach dem Entfernen der Sterilbarriere verbleibt dann dank des zusätzlichen Verschlusses ein erweitertes Zeitfenster für die Montage der betreffenden Komponente bzw. Separationseinheit(en).

Gemäß diesem Aspekt umfasst der Schritt des Schützens des Medium-berührenden Bereichs der gammasterilisierbaren Komponente und der gammasterilisierbaren Separationseinheit(en) und/oder der nicht-gammasterilisierbaren Separationseinheit(en) jeweils durch eine Sterilbarriere ein zusätzliches Abdecken des Medium-berührenden Bereichs der gammasterilisierbaren Komponente bzw. der gammasterilisierbaren Separationseinheit(en) mit wenigstens einem ersten Verschluss, und/oder ein zusätzliches Abdecken des Medium-berührenden Bereichs der nicht-gammasterilisierbaren Separationseinheit(en) mit wenigstens einem zweiten Verschluss. Der Schritt des Entfernens der Sterilbarrieren umfasst ein späteres Entfernen des ersten und/oder zweiten Verschlusses. Der Schritt des Montierens der gammasterilisierten Komponente bzw. der gammasterilisierten Separationseinheit(en) bzw. der Heißdampf-sterilisierten Separationseinheit(en) in der Einweg-Vorrichtung erfolgt unmittelbar nach dem Entfernen des ersten bzw. zweiten Verschlusses.

Die Bezeichnungen "erster" und "zweiter" Verschluss dienen hier lediglich zur Unterscheidung eines Verschlusses, der für die gammasterilisierbare Komponente vorgesehen ist, von einem Verschluss, der für die nicht-gammasterilisierbaren Untereinheit vorgesehen ist. Ein "erster" Verschluss setzt also nicht zwingend einen "zweiten" Verschluss voraus und umgekehrt.

Konkret kann der erweiterte Zeitraum zwischen dem Entfernen der jeweiligen Sterilbarriere und dem Montieren der zugehörigen, mit einem ersten Verschluss versehenen gammasterilisierten Komponente bzw. der gammasterilisierten Separationseinheit(en) bzw. der zugehörigen. mit einem zweiten Verschluss versehenen Heißdampf-sterilisierten Separationseinheit(en) jeweils kürzer als drei Stunden, vorzugsweise kürzer als zwei Stunden, weiter vorzugsweise kürzer als eine Stunde und weiter vorzugsweise kürzer als eine halbe Stunde sein.

Der Zeitraum zwischen dem Entfernen des ersten bzw. zweiten Verschlusses und dem Montieren der zugehörigen gammasterilisierten Komponente bzw. der gammasterilisierten Separationseinheit(en) bzw. Heißdampf-sterilisierten Separationseinheit(en) sollte aber wiederum jeweils kürzer als zwei Minuten, vorzugsweise kürzer als eine Minute und weiter vorzugsweise kürzer als eine halbe Minute sein.

Sofern zusätzlich zur Sterilbarriere zusätzlich wenigstens ein Verschluss zum Abdecken des Medium-berührenden Bereichs der gammasterilisierbaren Komponente bzw. der gammasterilisierten Separationseinheit(en) bzw. der nicht-gammasterilisierbaren Separationseinheit(en) vorgesehen ist, sollte ein solcher Verschluss für einen effektiven Schutz eine Partikeleintrag-reduzierende Barriere mit einem Spaltmaß von höchstens 100 µm, vorzugsweise von höchstens 50 µm und weiter vorzugsweise von höchstens 10 µm bilden. Grundsätzlich kann auch ein Verschluss verwendet werden, der selbst eine Sterilbarriere darstellt.

Beim erfindungsgemäßen Verfahren kann des Weiteren vorgesehen sein, dass der Schritt b) vor dem Schritt a) und/oder der Schritt d) vor dem Schritt c) erfolgt, d. h. die Medium-berührende Bereiche der gammasterilisierbaren Komponente bzw. der gammasterilisierten Separationseinheit(en) bzw. der nicht-gammasterilisierbaren bzw. der gammasterilisierten Separationseinheit(en) können schon vor dem Sterilisieren mit einer Sterilbarriere geschützt werden. Bei der gammasterilisierbaren Komponente bzw. der gammasterilisierten Separationseinheit(en) ist dementsprechend ein Gammastrahlen-resistentes Material für die Sterilbarriere zu wählen. Bei der wenigstens einen mit Heißdampf sterilisierten Separationseinheit(en) ist sicherzustellen, dass der Heißdampf in ausreichendem Maße durch die Sterilbarriere hindurchdringen kann. In beiden Fällen ist dann sichergestellt, dass nach dem Verbringen der sterilisierten Komponente bzw. Separationseinheit(en) in eine nicht-sterile Umgebung die Medium-berührenden Bereiche jeweils steril bleiben.

Als Sterilbarriere für die wenigstens eine Separationseinheit, die mit Heißdampf sterilisiert wird, eignet sich grundsätzlich eine die Separationseinheit vollständig umgebende, geschlossene Hülle, die aus einem Vliesstoff, vorzugsweise Tyvek^{®}, gebildet ist. Dieser Vliesstoff besteht aus Polyethylen hoher Dichte (PE-HD) und baut sich aus fibrillierten, eng miteinander zu Netzwerken verbundenen Feinstfilamenten im Durchmesserbereich von 0,5 bis 10 µm auf. Ein solches Material zeichnet sich durch seine hohe Wasserdampfdurchlässigkeit aus und erlaubt somit ein schnelles Eindringen von Heißdampf, welcher für die Sterilisierung der in der geschlossenen Hülle befindlichen wenigstens einen Separationseinheit erforderlich ist. Andererseits eignet sich das Material unter normalen Umgebungsbedingungen als Sterilbarriere. Das Material verliert nur sehr wenige Fasern, und es ist hydrophob, d. h. es zieht kein Wasser in das Material ein. Deshalb behält das Material seine Festigkeit, unabhängig davon, ob es mit Wasser benetzt oder trocken ist. In einer aus einem solchen Material gebildeten Hülle kann die wenigstens eine Separationseinheit also mit Heißdampf sterilisiert und anschließend gelagert und/oder transportiert werden.

Gemäß einem besonderen Aspekt der Erfindung wird der Medium-berührende Bereich der nicht-gammasterilisierbaren Separationseinheit(en) vor dem Sterilisieren mit Heißdampf und bis zum Montieren der Heißdampf-sterilisierten Separationseinheit(en) durch einen zweiten Verschluss, insbesondere einen Deckel, abgedeckt. Der Verschluss weist wenigstens eine Durchgangsöffnung auf, die selektiv freigegeben und verschlossen werden kann. Wie eingangs bereits erwähnt, kann dieser Verschluss die erfindungsgemäß vorgesehene Sterilbarriere für den Medium-berührenden Teil der Separationseinheit(en) bilden. Dies ist aber nicht zwingend notwendig, wenn die Sterilbarriere anderweitig, insbesondere durch die zuvor genannte Hülle gebildet ist.

Im Hinblick auf die bevorzugte Anwendung der Erfindung weist die wenigstens eine nicht-gammasterilisierbare Separationseinheit einen Fluidanschluss auf. Bei der Durchführung des erfindungsgemäßen Verfahrens wird der Fluidanschluss vorzugsweise durch einen zweiten Verschluss abgedeckt. Wenn zusätzlich eine Sterilbarriere vorgesehen ist, wie die zuvor genannte Hülle, kann nach deren Entfernung die Exposition des Fluidanschlusses verkürzt werden, indem der Verschluss erst unmittelbar vor der Montage der nicht-gammasterilisierbaren Separationseinheit abgenommen wird. Das bedeutet, dass die Zeitspanne, die für eine wahrscheinlich "reine" (nicht verunreinigte) Montage zur Verfügung steht, verlängert wird, da nach dem Entfernen der Sterilbarriere nicht innerhalb von zwei Minuten alle Fluidanschlüsse montiert werden müssen. Dies ist insbesondere bei komplizierten Aufbauten mit vielen Anschlüssen von Vorteil, da im Zuge der Montage ein Verschluss nach dem anderen entfernt werden kann.

In einer besonders bevorzugten Ausführungsform umfasst das erfindungsgemäße Verfahren folgende weitere Schritte bzw. Maßnahmen: Anbringen des zweiten Verschlusses auf dem Fluidanschluss der nicht-gammasterilisierbaren Separationseinheit(en); Freigeben der Durchgangsöffnung, falls die Durchgangsöffnung verschlossen ist; Verpacken der nicht-gammasterilisierbaren Separationseinheit(en) in die Hülle; Sterilisieren der verpackten Separationseinheit(en) mit Heißdampf bei freigegebener Durchgangsöffnung; nach dem Sterilisieren mit Heißdampf: Verschließen der Durchgangsöffnung in der geschlossenen Hülle; Auspacken der Heißdampf-sterilisierten Separationseinheit(en) aus der geschlossenen Hülle; und Abnehmen des zweiten Verschlusses unmittelbar vor dem Montieren der Heißdampf-sterilisierten Separationseinheit(en).

Das bedeutet, dass die Heißdampf-sterilisierten Separationseinheit(en) zuerst in einer Hülle verpackt und dann in der geschlossenen Hülle sterilisiert wird bzw. werden, sodass nach dem Sterilisieren keine Verunreinigung mehr in die Hülle gelangen kann. Dies ist möglich, da die Hülle - wie zuvor erläutert - aus einem Material hergestellt werden kann, das durchlässig für Heißdampf ist, unter normalen Bedingungen aber als Sterilbarriere wirkt.

Eine weitere Voraussetzung ist, dass sich die Separationseinheit(en) durch die Heißdampfsterilisierung nicht plastisch verformt bzw- verformen. Dies wird dadurch erreicht, dass die Durchgangsöffnung des vor der Sterilisierung angebrachten zweiten Verschlusses während der Heißdampfsterilisierung freigegeben ist, sodass durch den offenen Fluidanschluss der Separationseinheit ein Druckausgleich möglich ist.

Außerdem ist sichergestellt, dass nach dem Auspacken der Separationseinheit(en) aus der Hülle weiterhin eine Partikeleintrag-reduzierende Barriere besteht, die den Medium-berührenden Bereich vor Verunreinigungen schützt. Diese Barriere wird durch den zweiten Verschluss bereitgestellt, dessen Durchgangsöffnung nach dem Sterilisieren in der geschlossenen Hülle verschlossen wurde. Somit besteht nach dem Auspacken der Heißdampf-sterilisierten Separationseinheit(en) noch solange ein Schutz, bis der zweite Verschluss im Verlauf des Zusammenbaus der Einweg-Vorrichtung abgenommen wird.

Wie bereits erläutert wird gemäß der Erfindung die wenigstens eine gammasterilisierbare Komponente der Einweg-Vorrichtung separat, d. h. nicht zusammen mit der bzw den nicht-gammasterilisierbaren Separationseinheit(en) sterilisiert, da die Sterilisierung mit Heißdampf das Risiko birgt, dass danach die Maßhaltigkeit der Komponente nicht mehr gegeben ist. Gerade bei einem in dieser Hinsicht kritischem Bauteil, wie etwa dem erfindungsgemäß vorgesehenen starren Verbindungsrohr, an dessen Fluidanschlüsse mehrere, vorzugsweise in einer starren Halterung fixierte, Separationseinheiten und evtl. weitere Verbindungsrohre angeschlossen sind, ist die Maßhaltigkeit jedoch von essentieller Bedeutung. Diese wird bei der für die Komponente erfindungsgemäß vorgesehene Sterilisierung durch Gammastrahlung nicht beeinträchtigt.

Um bei der gammasterilisierten Komponente bzw. der gammasterilisierten Separationseinheit(en) der Einweg-Vorrichtung auch nach dem Entfernen der erfindungsgemäß vorgesehenen Sterilbarriere die Gefahr einer Verunreinigung des Medium-berührenden Bereichs der Komponente zu minimieren, kann vorgesehen sein, dass der Medium-berührende Bereich der gammasterilisierten Komponente bzw. der gammasterilisierten Separationseinheit(en) durch einen ersten Verschluss, vorzugsweise durch eine Blindkappe, einen Sterilkonnektor oder eine Umhüllung, abgedeckt bleibt. Da die Gammastrahlung die Maßhaltigkeit der Komponente bzw. der Separationseinheit(en) und des Verschlusses, sofern dieser aus einem geeigneten Material gebildet ist, weder kurz- noch langfristig beeinträchtigt (keine elastische oder plastische Verformung, keine Brüchigkeit o.ä.), kann der Verschluss bereits vor dem Sterilisieren angebracht werden und bis zum Montieren der Komponente bzw. der Separationseinheit(en) dort verbleiben. Somit besteht für den Medium-berührenden Bereich der gammasterilisierten Komponente bzw. der gammasterilisierten Separationseinheit(en) solange ein Schutz, bis der erste Verschluss im Zuge der Montage der Komponente abgenommen wird. Der erste Verschluss zum Abdecken des Medium-berührenden Bereichs der gammasterilisierbaren Komponente bzw. der gammasterilisierbaren Separationseinheit(en) kann aber im Einklang mit der Erfindung auch die alleinige Sterilbarriere für die Komponente bilden.

Im Hinblick auf die die bevorzugte Anwendung der Erfindung umfasst die wenigstens eine gammasterilisierbare Komponente typischerweise eine Mehrzahl von untereinander verbindbaren Verteilerbaugruppen zum Verbinden mehrerer Separationseinheiten und/oder wenigstens eine Anschlussschlauchleitung, wobei die gammasterilisierbare Komponente wenigstens einen Fluidanschluss aufweist, der wie zuvor beschrieben durch den ersten Verschluss abgedeckt wird.

Wenn kein erster Verschluss für die gammasterilisierbare Komponente bzw. die gammasterilisierbaren Separationseinheit(en) vorgesehen ist oder ein solcher Verschluss keine Sterilbarriere bildet, ist die erfindungsgemäß vorgesehene Sterilbarriere für den Medium-berührenden Teil der Komponente bzw. die gammasterilisierbaren Separationseinheit(en) vorzugsweise durch eine diese vollständig umgebende, geschlossene Primärverpackung gebildet.

Um das Risiko einer Verunreinigung aus der Umgebung weiter zu minimieren, sollten die Heißdampf-sterilisierte Separationseinheit(en) und die gammasterilisierte Komponente bzw. die gammasterilisierten Separationseinheit(en) auf einer Sicherheitswerkbank (Laminar Flow Werkbank, Cleanbench) oder in einem Reinraum, vorzugsweise in einem durch Wände abgetrennten Bereich eines Reinraums, montiert werden.

Zur Verwendung in einem erfindungsgemäßen Verfahren, bei dem ein zweiter Verschluss für eine Separationseinheit der Untereinheit vorgesehen ist, kann ein Deckel für einen Fluidanschluss der nicht-gammasterilisierbaren Separationseinheit(en) vorgesehen sein. Der Deckel umfasst einen Grundkörper zum passgenauen Anbringen des Deckels am Fluidanschluss. Der Deckel hat wenigstens eine Durchgangsöffnung, die nach dem Anbringen des Deckels am Fluidanschluss eine Strömungsverbindung zwischen dem Medium-berührenden Bereich der Separationseinheit und der unmittelbaren Umgebung der Separationseinheit bereitstellt. Der Deckel umfasst ferner einen Mechanismus zum selektiven Freigeben und Verschließen der wenigstens einen Durchgangsöffnung.

Ein solcher Deckel kann vor der Heißdampfsterilisierung am Fluidanschluss der Separationseinheit angebracht werden und bis zum Zusammenbau der Einweg-Vorrichtung dort verbleiben. Der besondere Verschlussmechanismus erlaubt es, dass der Fluidanschluss wahlweise mit der Umgebung in Strömungsverbindung gebracht oder von der Umgebung getrennt werden kann (zumindest um einem unerwünschten Partikeleintrag entgegenzuwirken), ohne dass der Deckel für das Freigeben und Verschließen vom Fluidanschluss abgenommen werden muss. Wie oben bereits erläutert, ist beim erfindungsgemäßen Verfahren vorgesehen, dass während der Sterilisierung mit Heißdampf die Durchgangsöffnung freigegeben ist, um einen Druckausgleich zu ermöglichen. Danach wird die Durchgangsöffnung verschlossen, um die gewünschte Partikelbarriere gegenüber der Umgebung zu bilden.

Gemäß einer bevorzugten Konstruktion des Deckels weist der Mechanismus einen Schließkörper auf, der zwischen einer Verschlussstellung, in der der Schließkörper die wenigstens eine Durchgangsöffnung abdeckt, und einer Öffnungsstellung überführbar ist, in der der Schließkörper die wenigstens eine Durchgangsöffnung nicht abdeckt. Das Abdecken und Freigeben erfolgt also nicht durch das Anbringen und Abnehmen des Deckels, sondern jeweils bei angebrachtem Deckel durch eine Bewegung des Schließkörpers.

Der Mechanismus zum Verschließen und Freigeben der Durchgangsöffnung sollte auch dann manuell betätigbar sein, wenn sich die Separationseinheit in der geschlossenen Hülle befindet. Hierfür weist der Deckel ein Betätigungselement auf, das leicht ertastbar und greifbar ist.

Im Gegensatz zur Separationseinheit selbst kann der Deckel eine wiederverwendbare Komponente sein, die vorzugsweise zumindest überwiegend aus Edelstahl gebildet ist. Wenn der Deckel nach dem Aufbau der Einweg-Vorrichtung nicht mehr benötigt wird, kann er nach entsprechender Reinigung für den gleichen Zweck bei einer anderen Einweg-Vorrichtung eingesetzt werden.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine perspektivische Ansicht einer Einweg-Vorrichtung zur Durchführung eines bioverfahrenstechnischen Prozesses;
- Figur 2 die Einweg-Vorrichtung aus Figur 1 in einer Draufsicht;
- Figur 3 eine Separationseinheit mit Anschlussschlauchleitungen und Konnektoren;
- Figur 4 einen Deckel für einen Fluidanschluss einer Separationseinheit;
- Figur 5 eine seitliche Explosionsansicht eines Verbindungsrohrs mit mehreren Fluidanschlüssen; und
- Figur 6 eine Draufsicht auf das Verbindungsrohr aus Figur 5.

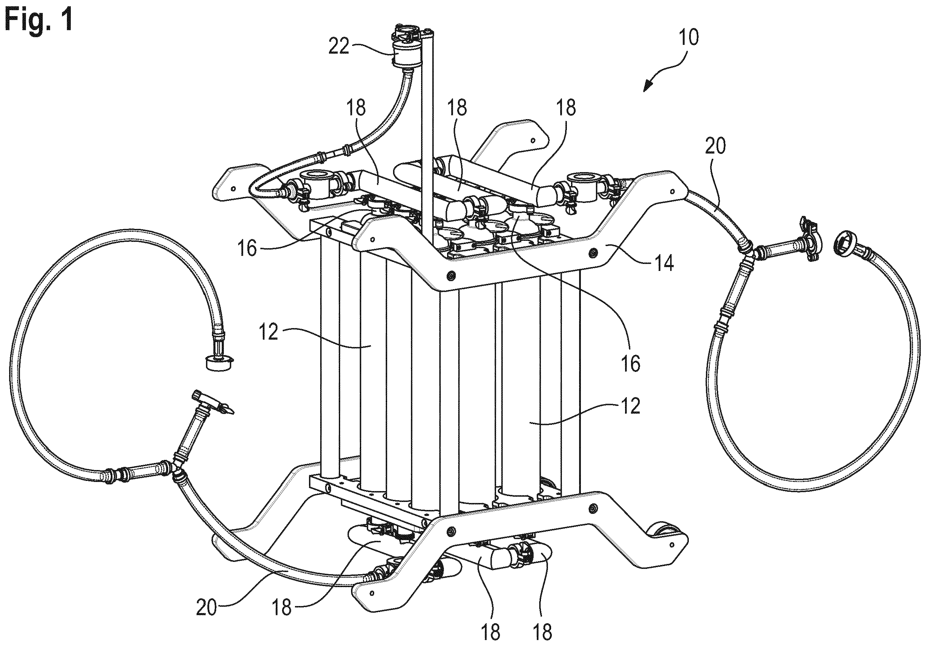

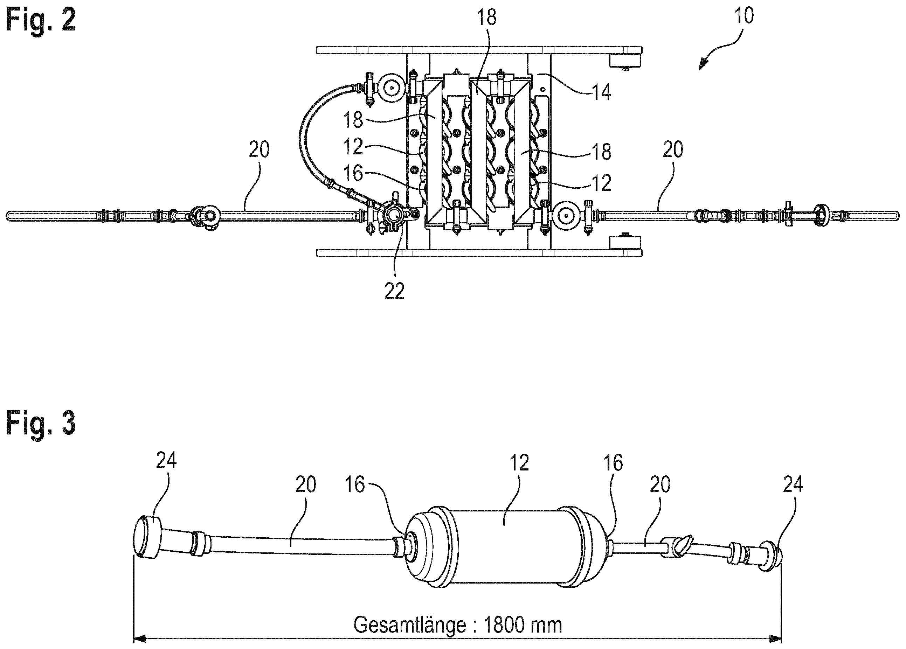

In den Figuren 1 und 2 ist eine Einweg-Vorrichtung 10 zur Durchführung eines bioverfahrenstechnischen Prozesses im zusammengebauten Zustand gezeigt.

Die Einweg-Vorrichtung 10 umfasst mehrere Separationseinheiten 12, die in einer starren Halterung 14 in einem vorgegebenen Raster angeordnet sind. Bei den Separationseinheiten 12 kann es sich grundsätzlich um Filtercapsulen, Chromatographiesäulen oder Membranadsorber handeln.

Die Fluidanschlüsse (Ein- und Ausgänge) 16 der Separationseinheiten 12 sind über starre Verbindungsrohre 18 oder Schlauchleitungen miteinander verschaltet. An die Verbindungsrohre 18 sind zum Zuführen und Abführen von Medium Anschlussschlauchleitungen 20 angeschlossen.

Außerdem ist an ein oberes Verbindungsrohr 18 ein Steril-Luftfilter 22 zum gemeinsamen Entlüften aller Separationseinheiten 12 angeschlossen.

Die Einweg-Vorrichtung 10 kann noch weitere Komponenten aufweisen. Der genaue Aufbau und die Betriebsweise der Einweg-Vorrichtung 10 sind für die hier beschriebene Erfindung jedoch nicht von wesentlicher Bedeutung.

Bei der Einweg-Vorrichtung 10 handelt es sich um eine "große" Vorrichtung für den Einsatz in der kommerziellen Fertigung mit einem großen Mediumdurchsatz (Produktionsmaßstab). Bei solchen Vorrichtungen sind mehrere Separationseinheiten miteinander verbunden, insbesondere mehr als drei 30-Zoll-Filtercapsulen (Länge ca. 850 mm). Davon abzugrenzen sind kleinere Vorrichtungen, die in Labors für experimentelle Zwecke bzw. zur Entwicklung von Prozessen verwendet werden (Labormaßstab).

Die Einweg-Vorrichtung 10 soll als Ganzes und, soweit möglich, in sterilem Zustand oder zumindest mit einer möglichst geringen Keimbelastung (Bioburden) und sonstigen Verunreinigungen durch Partikel o.ä. an den Anwender ausgeliefert werden. Grundsätzlich ist in diesem Zusammenhang eine Sterilisierung durch Gammastrahlung bevorzugt, da bei diesem Verfahren in der Regel die Maßhaltigkeit der Komponenten gewährleistet werden kann.

Die Einweg-Vorrichtung 10 enthält im vorliegenden Fall jedoch wenigstens eine Separationseinheit 12, die nicht für eine Sterilisierung durch Gammastrahlung geeignet ist. Dies ist insbesondere dann der Fall, wenn das Filter- bzw. das Membranmaterial durch die bei einer Gammasterilisierung typischerweise verabreichte Energiedosis (z. B. 25 kGy) so stark beschädigt würde, dass eine bestimmungsgemäße Funktion nicht mehr garantiert werden kann. Beispiele für solche ungeeigneten Materialien sind Polytetrafluorethylen (PTFE), Polypropylen (PP) und Polyvinylchlorid (PVC).

Die nicht durch Gammastrahlung sterilisierbaren Separationseinheiten 12 werden als Untereinheiten einzeln oder in einem Verbund, ggf. mit weiteren Komponenten, mit Heißdampf in einem Autoklav sterilisiert. Eine solche Untereinheit darf nur so groß sein, dass sie im Autoklav untergebracht werden kann.

Bei der Einteilung der Einweg-Vorrichtung 10 in Untereinheiten ist neben den äußeren Abmessungen noch ein weiteres Kriterium zu beachten: Es muss sichergestellt sein, dass bei der Behandlung im Autoklav vor allem die inneren Flächen der Separationseinheiten 12 in ausreichendem Maße und lange genug mit dem Heißdampf in Berührung kommen. Dies ist insbesondere dann von Bedeutung, wenn mehrere Separationseinheiten 12 miteinander verbunden sind, wie etwa durch die Verbindungsrohre 18, oder an Schlauchleitungen 20 angeschlossen sind, sodass der Heißdampf nicht direkt durch die Fluidanschlüsse 16 in die Separationseinheiten 12 eindringen kann. Es besteht dann grundsätzlich die Gefahr, dass der Heißdampf nicht alle relevanten Flächen erreicht bzw. nicht schnell genug erreicht, um eine ausreichende Sterilisierung zu gewährleisten.

In der Praxis hat sich gezeigt, dass die vom Heißdampf zu durchströmende Gesamtlänge einer Untereinheit 1800 mm nicht überschreiten sollte. Dies ist in Figur 3 anhand eines Beispiels gezeigt, bei dem die zu durchströmende Gesamtlänge einer Filtercapsule 12 mit zwei angeschlossenen Schlauchleitungen 20 und Konnektoren 24 an den freien Enden etwa 1800 mm beträgt.

Nachfolgend wird anhand eines bevorzugten Ausführungsbeispiels beschrieben, wie eine vorgegebene Reinheit, insbesondere eine vorgegebene mikrobiologische Reinheit, der Einweg-Vorrichtung 10 bis zur Inbetriebnahme der gesamten Einweg-Vorrichtung 10 bei einem Anwender sichergestellt wird.

Zunächst wird die Heißdampfsterilisierung einer Untereinheit mit einer Separationseinheit 12 beschrieben. Diese Beschreibung ist ggf. auf die weiteren Separationseinheiten 12 derselben Untereinheit bzw. auf die Separationseinheiten 12 weiterer Untereinheiten zu übertragen.

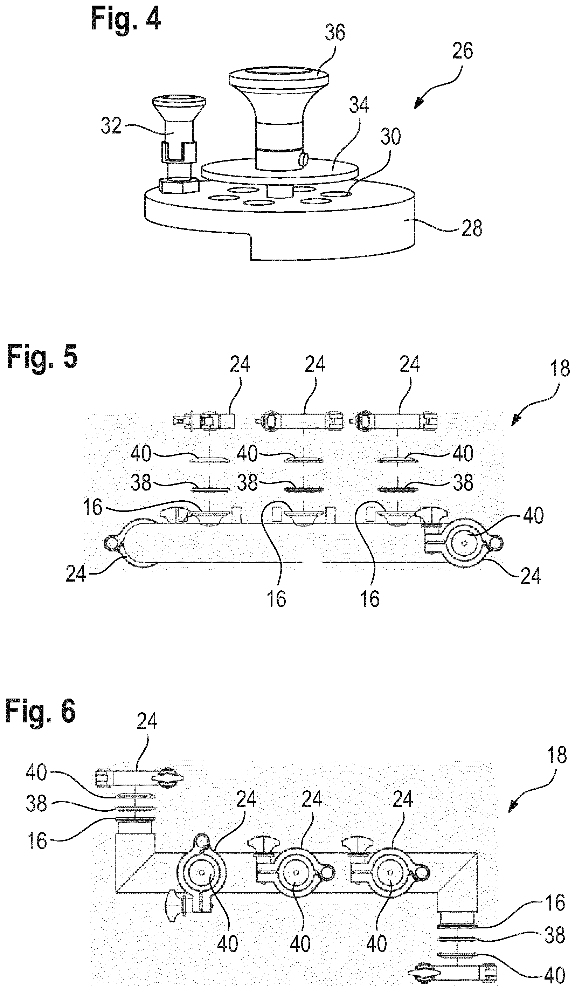

Diejenigen Stirnseiten der Separationseinheit 12, an denen sich ein oder mehrere freie Fluidanschlüsse 16 befinden (d. h. Fluidanschlüsse 16, an denen vor der Sterilisierung kein Verbindungsrohr 18 und keine Schlauchleitung 20 o.ä. angeschlossen ist), werden mit einem Verschluss versehen. Bei dem hier beschriebenen Ausführungsbeispiel kommt als Verschluss ein Deckel 26 zum Einsatz, wie er in Figur 4 gezeigt ist. Der überwiegend aus Edelstahl gebildete Deckel 26 ist eine Mehrweg-Komponente, d. h. er kann für weitere Sterilisierungsvorgänge verwendet werden.

Der Deckel 26 besteht im Wesentlichen aus einem Grundkörper 28 mit einer oder mehreren durchgehenden Durchgangsöffnungen 30, einem Arretierbolzen 32 und einem Verschlussmechanismus zum selektiven Freigeben und Verschließen der Durchgangsöffnungen 30.

Der Grundkörper 28 ist genau an die Außenkontur der zugeordneten Stirnseite der Separationseinheit 12 angepasst und kann mittels des Arretierbolzens 32 an der Separationseinheit 12 fixiert werden.

Der Verschlussmechanismus weist einen Schließkörper 34 auf, der zwei definierte, stabile Stellungen einnehmen kann: eine Verschlussstellung, in der der Schließkörper 34 fest am Grundkörper 28 anliegt und die Durchgangsöffnungen 30 abdeckt, und eine Öffnungsstellung, in der der Schließkörper 34 angehoben ist, sodass er die Durchgangsöffnungen 30 nicht abdeckt.

Der Verschlussmechanismus ist mittels eines Betätigungselements 36 manuell betätigbar, d. h. durch Drücken bzw. Ziehen am Betätigungselement 36 kann der Schließkörper 34 in die jeweils andere Stellung überführt werden.

In der fixierten Position des Deckels 26 stehen der bzw. die Fluidanschlüsse 16 der Separationseinheit 12 über die Durchgangsöffnungen 30 in Strömungsverbindung mit der unmittelbaren Umgebung der Separationseinheit 12, wenn sich der Schließkörper 34 in der Öffnungsstellung befindet. Befindet sich der Schließkörper 34 dagegen in der Verschlussstellung, besteht keine, bzw. so gut wie keine Strömungsverbindung zwischen dem Inneren der Separationseinheit 12 und der Umgebung, d h. ein möglicher Partikeleintrag ist weitgehend verhindert. Der Grundkörper 28 und der Schließkörper 34 sind so ausgelegt, dass der Deckel 26 in der Verschlussstellung ein Spaltmaß von höchstens 100 µm, vorzugsweise von höchstens 50 µm und weiter vorzugsweise von höchstens 10 µm, sodass entsprechend größere Partikel abgehalten werden.

Nach dem Anbringen des Deckels 26 auf der Stirnseite der Separationseinheit 12 (und ggf. eines weiteren Deckels 26 auf der anderen Stirnseite) wird die Untereinheit in eine Hülle gepackt, wobei vorher sichergestellt wird, dass die Durchgangsöffnungen 30 freigegeben sind. Die Hülle besteht aus Tyvek^{®} oder einem vergleichbaren Material, das einerseits wasserdicht ist und unter normalen Umgebungsbedingungen als Sterilbarriere dienen kann, andererseits aber durchlässig für Wasserdampf ist. Die Hülle wird durch Schweißen geschlossen.

Anschließend wird die Untereinheit in der Hülle in den Autoklav eingelegt und unter vorgegebenen Bedingungen mit Heißdampf sterilisiert (z. B. 40 min bei 121 °C oder 30 min bei 131 °C). Der Heißdampf gelangt durch die Hülle hindurch an die Außenflächen der Untereinheit und durch die freigegebenen Durchgangsöffnungen 30 in das Innere der Separationseinheit 12. Die Durchgangsöffnungen 30 sind hierfür ausreichend groß dimensioniert. Die Durchgangsöffnungen 30 sorgen während der Heißdampfsterilisierung auch dafür, dass ein ausreichender Druckausgleich stattfinden kann, sodass sich die Separationseinheit 12 nicht plastisch verformt.

Die Hülle bleibt nach der Sterilisierung geschlossen und dient bis zum Zusammenbau der Einweg-Vorrichtung 10 als Sterilbarriere für die Untereinheit.

Die übrigen Komponenten der Einweg-Vorrichtung 10 werden vor deren Zusammenbau durch Gammastrahlung sterilisiert. Vor der Bestrahlung werden die offenen Fluidanschlüsse 16 der Verbindungsrohre 18 und der Anschlussschlauchleitungen 20 ebenfalls mit Verschlüssen abgedeckt, hier in Form von Dichtungen 38 und Blindkappen 40, die mittels Konnektoren 24, wie etwa Tri-Clamp-Klemmverbindern, angebracht werden. Dies ist am Beispiel eines Z-förmigen Verbindungsrohrs 18 in den Figuren 5 und 6 gezeigt. Die Verschlüsse und etwaige Hilfsmittel zu deren Befestigung bestehen aus Gammastrahlenresistentem Material und sind so ausgelegt, dass sie einen Partikeleintrag durch die Fluidanschlüsse 16 effektiv verhindern, wobei die Spaltmaße vergleichbar zu den bevorzugten Spaltmaßen bei dem durch den Deckel 26 abgedeckten Fluidanschluss 16 der Separationseinheit 12 sind. Grundsätzlich können die Verschlüsse auch als Sterilbarrieren ausgelegt sein.

Danach werden die Verbindungsrohre 18 und Anschlussschlauchleitungen 20 in eine Primärverpackung verpackt, die als Sterilbarriere dient. Die Sterilisierung dieser Komponenten erfolgt dann im verpackten Zustand, weshalb für die Primärverpackung ebenfalls ein Material zu wählen ist, dessen Eigenschaften durch Gammastrahlung in der für eine Sterilisierung üblichen Dosis nicht wesentlich beeinträchtigt werden.

Der Zusammenbau der Einweg-Vorrichtung 10 erfolgt entweder beim Hersteller oder - nach Transport der steril verpackten Untereinheiten und weiteren Komponenten - beim Anwender, und vorzugsweise auf einer Sicherheitswerkbank, die ein steriles Konnektieren ermöglicht, oder in einem durch Wände abgetrennten Bereich eines Reinraums.

Vor dem Auspacken der Untereinheit aus der Hülle werden die Durchgangsöffnungen 30 des Deckels 26 verschlossen. Dies geschieht bei geschlossener Hülle durch Ergreifen des Betätigungselements 36 und Betätigen des Verschlussmechanismus. Trotz der dazwischen befindlichen Hülle kann das markante Betätigungselement 36 durch Ertasten leicht ausfindig gemacht werden.

Durch das Verschließen der Durchgangsöffnungen 30 bildet der Deckel 26 auch nach dem Auspacken der Untereinheit einen Schutz vor Partikeleintrag, ggf. sogar eine Sterilbarriere, sodass ein Eindringen von Verunreinigungen in das Innere der Separationseinheit 12 weitgehend vermieden wird.

Ebenso dienen die Blindkappen 40 nach dem Auspacken als Schutz vor Partikeleintrag, bevorzugt sogar als Sterilbarrieren, und verhindern somit weitgehend, dass Verunreinigungen in das Innere der Verbindungsrohre 18 bzw. der Anschlussschlauchleitungen 20 eindringen können.

Dennoch sollte beim Zusammenbau der Einweg-Vorrichtung 10 die Hülle bzw. die Primärverpackung jeweils so spät wie möglich geöffnet werden, um die Exposition der mit den Verschlüssen abgedeckten Fluidanschlüsse 16 gegenüber der Umgebung so kurz wie möglich zu halten. Die Exposition kann aber dank der Verschlüsse insgesamt bis zu drei Stunden betragen. Bevorzugt ist jedoch weniger als zwei Stunden, noch besser weniger als eine Stunde und idealerweise weniger als eine halbe Stunde.

In dieser Zeit wird jede Separationseinheit 12 an ihren vorgesehenen Platz in der starren Halterung 14 gestellt. Die Verbindungsrohre 18 und Anschlussschlauchleitungen 20, soweit nicht bereits vormontiert, werden bereitgelegt. Erst unmittelbar vor dem Anschließen einer Komponente werden die Deckel 26 bzw. Blindkappen 40 von den für die jeweilige Verbindung benötigten Fluidanschlüssen 16 abgenommen. Nach dem Abnehmen eines Deckels 26 bzw. einer Blindkappe 40 sollte die Exposition des jeweiligen offenen Fluidanschlusses 16 gegenüber der Umgebung deutlich kürzer gehalten werden. Bevorzugt beträgt diese Zeitspanne weniger als zwei Minuten, besser weniger als eine Minute und idealerweise weniger als 30 Sekunden. Eine Montage in einer Reinraumklasse besser als ISO 8 ist unter diesen Voraussetzungen dann nicht zwingend erforderlich.

Üblicherweise werden auf die oben beschriebene Weise zunächst die Verbindungsrohre 18 an die Separationseinheiten 12 angeschlossen, bevor dann die Anschlussschlauchleitungen 20 an die Verbindungsrohre 18 angeschlossen werden. In jedem Fall ist am Ende ein in sich geschlossenes System hergestellt.

Sofern der Zusammenbau bereits beim Hersteller, also nicht beim späteren Anwender, erfolgt, wird die zusammengebaute Einweg-Vorrichtung 10 als Ganzes in eine als Sterilbarriere dienende Verpackung verpackt und an den Anwender ausgeliefert.

Nach dem Aufstellen bzw. Zusammenbau der Einweg-Vorrichtung 10 beim Anwender verbleiben die Blindkappen 40 an den freien Schlauchenden solange dort, bis die Anschlussschlauchleitungen 20 im Rahmen der Inbetriebnahme an eine Mediumzufuhr bzw. -abfuhr angeschlossen werden.

Als weitere Sicherheitsmaßnahmen sind für die oben beschriebenen Arbeitsschritte beim Zusammenbau der Einweg-Vorrichtung 10 das Anlegen steriler Handschuhe und eines Mundschutzes vorgesehen. Die ansonsten übliche Reinraumkleidung ist obligatorisch.

Für den Zusammenbau der Einweg-Vorrichtung 10 sind zwei Personen vorgesehen, insbesondere um die gewünschte kurze Exposition offener Fluidanschlüsse 16 zu erreichen. Die erste Person ist für das Auspacken und die Bereitstellung der Komponenten zuständig. Die zweite Person nimmt die Deckel 26 und die Blindkappen 40 von den Fluidanschlüssen 16 ab und stellt die vorgesehenen Strömungsverbindungen her, d. h. sie arbeitet vornehmlich an den Stellen, die kritisch für die Reinheit im Inneren der Medium-durchströmten Komponenten sind.

Zudem werden Arbeitsflächen, Werkezuge und Ablagebehälter gesondert gereinigt.

Die Einweg-Vorrichtung 10 mit deren Separationseinheiten und übrigen Komponenten wurde nur beispielhaft beschrieben. Zusätzlich oder alternativ zu den Separationseinheiten 12 können auch andere Funktionseinheiten vorgesehen sein, die nicht für eine Sterilisierung durch Gammastrahlung geeignet sind, wie etwa Sensoranordnungen mit elektronischen Datenspeichern, die durch Gammastrahlung beeinträchtigt würden.

Anstelle des beschriebenen Deckels 26 kann für die Separationseinheiten 16 bzw. anderen Funktionseinheiten auch ein anderer Verschluss mit vergleichbarer Funktionalität verwendet werden. Ein Beispiel hierfür ist ein Stopfen aus Silikon oder einem ähnlichen Material mit einem oder mehreren Schlitzen, der auf einem Fluidanschluss 16 einer Separationseinheit 12 angebracht wird. Beim Erhitzen im Autoklav dehnt sich der Stopfen aus, und die Schlitze bilden Durchgangsöffnungen, sodass Heißdampf durch die so gebildeten Durchgangsöffnungen in das Innere der Separationseinheit 12 gelangen kann. Beim anschließenden Abkühlen schrumpft der Stopfen wieder auf seine Normalgröße, sodass sich die Durchgangsöffnungen schließen und ein Eindringen von Verunreinigungen durch die Schlitze weitgehend vermieden wird. Es ist auch möglich, einen Luft- bzw. Heißdampf-durchlässigen Staubbeutel als Schutz um den Fluidanschluss 16 zu legen und dort zu fixieren.

Zum (selektiven) Verbinden der Separationseinheiten 12 bzw. Funktionseinheiten können auch mehrere direkt miteinander verbindbare, gammasterilisierbare Verteilerbaugruppen verwendet werden, die direkt an den Stirnseiten der Separationseinheiten 12 bzw. Funktionseinheiten angebracht werden.

### Bezugszeichenliste

- 10: Einweg-Vorrichtung
- 12: Separationseinheit
- 14: Halterung
- 16: Fluidanschluss
- 18: Verbindungsrohr
- 20: (Anschluss-)Schlauchleitung
- 22: Steril-Luftfilter
- 24: Konnektor
- 26: Deckel
- 28: Grundkörper
- 30: Durchgangsöffnung
- 32: Arretierbolzen
- 34: Schließkörper
- 36: Betätigungselement
- 38: Dichtung
- 40: Blindkappe

## Patentansprüche

1. Verfahren zur Sicherstellung einer mikrobiologischen Reinheit einer großen Einweg-Vorrichtung (10) zur Durchführung eines bioverfahrenstechnischen Prozesses in der kommerziellen Fertigung mit einem großen Mediumdurchsatz, wobei die Einweg-Vorrichtung (10)
wenigstens eine gammasterilisierbare Komponente, die aus für eine Sterilisierung durch Gammastrahlung geeigneten Materialien gebildet ist, und
mehrere Separationseinheiten in Form von Filtercapsulen, Chromatographiesäulen oder Membranadsorbern umfasst, von denen wenigstens eine ein für eine Sterilisierung durch Gammastrahlung ungeeignetes Material enthält und somit nicht-gammasterilisierbar ist,
wobei sowohl die Komponente als auch die Separationseinheiten jeweils einen Bereich aufweisen, der bei der Durchführung des bioverfahrenstechnischen Prozesses mit einem Prozess-Medium in Berührung kommt, und
wobei das Verfahren folgende Schritte umfasst:
a) Sterilisieren der gammasterilisierbaren Komponente und der gammasterilisierbaren Separationseinheit(en) durch Gammastrahlung;
b) Schützen des Medium-berührenden Bereichs der gammasterilisierbaren Komponente und der gammasterilisierbaren Separationseinheit(en) jeweils durch eine Sterilbarriere;
c) Sterilisieren der nicht-gammasterilisierbaren Separationseinheit(en) als Untereinheit einzeln oder in einem Verbund mit Heißdampf;
d) Schützen des Medium-berührenden Bereichs der nicht-gammasterilisierbaren Separationseinheit(en) jeweils durch eine Sterilbarriere;
e) Entfernen der Sterilbarrieren; und
f) Montieren der gammasterilisierten Komponente und der gammasterilisierten Separationseinheit(en) sowie der Heißdampf-sterilisierten Separationseinheit(en) in der Einweg-Vorrichtung (10) unmittelbar nach dem Entfernen der Sterilbarrieren,
wobei die wenigstens eine gammasterilisierbare Komponente wenigstens ein starres Verbindungsrohr (18) mit Fluidanschlüssen umfasst, an denen nach Zusammenbau der Einweg-Vorrichtung (10) mehrere Separationseinheiten angeschlossen sind.

2. Verfahren nach Anspruch 1, **dadurch gekennzeichnet, dass** der Zeitraum zwischen dem Entfernen einer Sterilbarriere und dem Montieren der zugehörigen gammasterilisierten Komponente bzw. der gammasterilisierten Separationseinheit(en) bzw. der zugehörigen Heißdampf-sterilisierten Separationseinheit(en) jeweils kürzer als vier Minuten, vorzugsweise kürzer als zwei Minuten, weiter vorzugsweise kürzer als eine Minute und noch weiter vorzugsweise kürzer als eine halbe Minute ist.

3. Verfahren nach Anspruch 1, **dadurch gekennzeichnet,**
**dass** der Schritt des Schützens des Medium-berührenden Bereichs der gammasterilisierbaren Komponente und der gammasterilisierbaren Separationseinheit(en) und/oder der nicht-gammasterilisierbaren Separationseinheit(en) jeweils durch eine Sterilbarriere ein zusätzliches Abdecken des Medium-berührenden Bereichs der gammasterilisierbaren Komponente bzw. der gammasterilisierbaren Separationseinheit(en) mit wenigstens einem ersten Verschluss, und/oder ein zusätzliches Abdecken des Medium-berührenden Bereichs der nicht-gammasterilisierbaren Separationseinheit(en) mit wenigstens einem zweiten Verschluss umfasst,
**dass** der Schritt des Entfernens der Sterilbarrieren ein späteres Entfernen des ersten und/oder zweiten Verschlusses umfasst, und
**dass** der Schritt des Montierens der gammasterilisierten Komponente bzw. der gammasterilisierten Separationseinheit(en) bzw. der Heißdampf-sterilisierten Separationseinheit(en) in der Einweg-Vorrichtung (10) unmittelbar nach dem Entfernen des ersten bzw. zweiten Verschlusses erfolgt.

4. Verfahren nach Anspruch 3, **dadurch gekennzeichnet,**
**dass** der Zeitraum zwischen dem Entfernen der jeweiligen Sterilbarriere und dem Montieren der zugehörigen, mit einem ersten Verschluss versehenen gammasterilisierten Komponente bzw. der gammasterilisierten Separationseinheit(en) bzw. der zugehörigen, mit einem zweiten Verschluss versehenen Heißdampf-sterilisierten Separationseinheit(en) jeweils kürzer als drei Stunden, vorzugsweise kürzer als zwei Stunden, weiter vorzugsweise kürzer als eine Stunde und weiter vorzugsweise kürzer als eine halbe Stunde ist, und
**dass** der Zeitraum zwischen dem Entfernen des ersten bzw. zweiten Verschlusses und dem Montieren der zugehörigen gammasterilisierten Komponente bzw. der gammasterilisierten Separationseinheit(en) bzw. der Heißdampf-sterilisierten Separationseinheit(en) jeweils kürzer als zwei Minuten, vorzugsweise kürzer als eine Minute und weiter vorzugsweise kürzer als eine halbe Minute ist.

5. Verfahren nach Anspruch 3 oder 4, **dadurch gekennzeichnet, dass** der erste und/oder der zweite Verschluss eine Partikeleintrag-reduzierende Barriere mit einem Spaltmaß von höchstens 100 µm, vorzugsweise von höchstens 50 µm, weiter vorzugsweise von höchstens 10 µm bildet.

6. Verfahren nach einem der vorhergehenden Ansprüche, **dadurch gekennzeichnet, dass** der Schritt b) vor dem Schritt a) und/oder der Schritt d) vor dem Schritt c) erfolgt.

7. Verfahren nach einem der vorhergehenden Ansprüche, **dadurch gekennzeichnet, dass** die Sterilbarriere für die Heißdampf-sterilisierte Separationseinheit(en) eine die Heißdampf-sterilisierten Separationseinheit(en) vollständig umgebende, geschlossene Hülle umfasst.

8. Verfahren nach Anspruch 7, **dadurch gekennzeichnet, dass** die Hülle aus einem wasserdichten, aber wasserdampfdurchlässigen Vliesstoff gebildet ist.

9. Verfahren nach einem der vorhergehenden Ansprüche, **dadurch gekennzeichnet, dass** der Medium-berührende Bereich der nicht-gammasterilisierbaren Separationseinheit(en) vor dem Sterilisieren mit Heißdampf und bis zum Montieren der Heißdampf-sterilisierten Separationseinheit(en) durch einen zweiten Verschluss, insbesondere einen Deckel (26), abgedeckt ist, wobei der zweite Verschluss wenigstens eine Durchgangsöffnung (30) aufweist, die selektiv freigegeben und verschlossen werden kann.

10. Verfahren nach einem der vorhergehenden Ansprüche, **dadurch gekennzeichnet, dass** die wenigstens eine nicht-gammasterilisierbare Separationseinheit einen Fluidanschluss (16) aufweist, der durch einen zweiten Verschluss abgedeckt wird.

11. Verfahren nach Anspruch 10, **gekennzeichnet durch** folgende Schritte bzw. Maßnahmen:
- Anbringen des zweiten Verschlusses auf dem Fluidanschluss (16) der nicht-gammasterilisierbaren Separationseinheit(en);
- Freigeben der Durchgangsöffnung (30), falls die Durchgangsöffnung (30) verschlossen ist;
- Verpacken der nicht-gammasterilisierbaren Separationseinheit(en) in die Hülle;
- Sterilisieren der verpackten Separationseinheit(en) mit Heißdampf bei freigegebener Durchgangsöffnung (30);
- nach dem Sterilisieren mit Heißdampf: Verschließen der Durchgangsöffnung (30) in der geschlossenen Hülle;
- Auspacken der Heißdampf-sterilisierten Separationseinheit(en) aus der geschlossenen Hülle; und
- Abnehmen des zweiten Verschlusses unmittelbar vor dem Montieren der Heißdampf-sterilisierten Separationseinheit(en).

12. Verfahren nach einem der vorhergehenden Ansprüche, **dadurch gekennzeichnet, dass** der Medium-berührende Bereich der gammasterilisierbaren Komponente bzw. der gammasterilisierbaren Separationseinheit(en) vor dem Sterilisieren durch Gammastrahlung und bis zum Montieren der gammasterilisierten Komponente bzw. der gammasterilisierten Separationseinheit(en) durch einen ersten Verschluss, vorzugsweise durch eine Blindkappe (40), einen Sterilkonnektor oder eine Umhüllung, abgedeckt bleibt.

13. Verfahren nach Anspruch 12, **dadurch gekennzeichnet, dass** die gammasterilisierbare Komponente eine Mehrzahl von untereinander verbindbaren Verteilerbaugruppen zum Verbinden mehrerer Separationseinheiten (12) und/oder wenigstens eine Anschlussschlauchleitung (20) umfasst, wobei die gammasterilisierbare Komponente wenigstens einen Fluidanschluss (16) aufweist, der durch den ersten Verschluss abgedeckt wird.

14. Verfahren nach einem der vorhergehenden Ansprüche, **dadurch gekennzeichnet, dass** die Sterilbarriere für die gammasterilisierbare Komponente bzw. die gammasterilisierbaren Separationseinheit(en) eine diese vollständig umgebende, geschlossene Primärverpackung umfasst.

15. Verfahren nach einem der vorhergehenden Ansprüche, **dadurch gekennzeichnet, dass** die Heißdampf-sterilisierte Separationseinheit(en) sowie die gammasterilisierte Komponente und die gammasterilisierbaren Separationseinheit(en) auf einer Sicherheitswerkbank oder in einem Reinraum, vorzugsweise in einem durch Wände abgetrennten Bereich eines Reinraums, montiert werden.

## Claims

1. Method of ensuring a microbiological purity of a large single-use device (10) for carrying out a biotechnological process in commercial manufacturing involving a large throughput of medium, wherein the single-use device (10) comprises
at least one gamma-sterilizable component which is formed from materials suitable for a sterilization by gamma radiation, and
a plurality of separation units in the form of filter capsules, chromatography columns or membrane adsorbers, at least one of which contains a material unsuitable for a sterilization by gamma radiation and thus is non-gamma-sterilizable,
wherein both the component and the separation units each have an area that comes into contact with a process medium when the biotechnological process is carried out, and
wherein the method comprises the following steps:
a) sterilizing the gamma-sterilizable component and the gamma-sterilizable separation unit(s) by gamma radiation;
b) protecting the medium-contacting area of the gamma-sterilizable component and of the gamma-sterilizable separation unit(s) in each case by a sterile barrier;
c) sterilizing the non-gamma-sterilizable separation unit(s) as a subunit, either individually or in combination, with superheated steam;
d) protecting the medium-contacting area of the non-gamma-sterilizable separation unit(s) in each case by a sterile barrier;
e) removing the sterile barriers; and
f) mounting the gamma-sterilized component and the gamma-sterilized separation unit(s) and also the superheated steam-sterilized separation unit(s) in the single-use device (10) immediately after removing the sterile barriers,
wherein the at least one gamma-sterilizable component comprises at least one rigid connecting pipe (18) having fluid connections, to which a plurality of separation units are connected following assembly of the single-use device (10).

2. Method according to claim **1, characterized in that** the time period between removing a sterile barrier and mounting the associated gamma-sterilized component and/or the gamma-sterilized separation unit(s) and/or the associated superheated steam-sterilized separation unit(s) is in each case shorter than four minutes, preferably shorter than two minutes, more preferably shorter than one minute, and yet more preferably shorter than half a minute.

3. Method according to claim **1, characterized in that** the step of protecting the medium-contacting area of the gamma-sterilizable component and of the gamma-sterilizable separation unit(s) and/or of the non-gamma-sterilizable separation unit(s) in each case by a sterile barrier comprises additionally covering the medium-contacting area of the gamma-sterilizable component and/or of the gamma-sterilizable separation unit(s) with at least one first closure, and/or additionally covering the medium-contacting area of the non-gamma-sterilizable separation unit(s) with at least one second closure,
**in that** the step of removing the sterile barriers comprises later removing the first and/or second closure, and
**in that** the step of mounting the gamma-sterilized component and/or the gamma-sterilizable separation unit(s) and/or the superheated steam-sterilized separation unit(s) in the single-use device (10) takes place immediately after removing the first and/or second closure.

4. Method according to claim **3, characterized**
**in that** the time period between removing the respective sterile barrier and mounting the associated gamma-sterilized component and/or gamma-sterilized separation unit(s) provided with a first closure and/or the associated superheated steam-sterilized separation unit(s) provided with a second closure is in each case shorter than three hours, preferably shorter than two hours, more preferably shorter than one hour, and yet more preferably shorter than half an hour, and
**in that** the time period between removing the first and/or second closure and mounting the associated gamma-sterilized component and/or the gamma-sterilized separation unit(s) and/or the superheated steam-sterilized separation unit(s) is in each case shorter than two minutes, preferably shorter than one minute, and more preferably shorter than half a minute.

5. Method according to claim 3 or 4, **characterized in that** the first and/or the second closure forms a particle ingress-reducing barrier having a gap size of at most 100 µm, preferably of at most 50 µm, and more preferably of at most 10 µm.

6. Method according to any one of the preceding claims, **characterized in that** step b) takes place before step a), and/or step d) takes place before step c).

7. Method according to any one of the preceding claims, **characterized in that** the sterile barrier for the superheated steam-sterilized separation unit(s) comprises a closed envelope which completely surrounds the superheated steam-sterilized separation unit(s).

8. Method according to claim 7, **characterized in that** the envelope is formed from a watertight but water vapor-permeable nonwoven fabric.

9. Method according to any one of the preceding claims, **characterized in that** the medium-contacting area of the non-gamma-sterilizable separation unit(s) is covered by a second closure, in particular a lid (26), prior to being sterilized with superheated steam and until the superheated steam-sterilized separation unit(s) are mounted, wherein the second closure has at least one passage opening (30) which can be selectively uncovered and closed.

10. Method according to any one of the preceding claims, **characterized in that** the at least one non-gamma-sterilizable separation unit has a fluid connection (16) which is covered by a second closure.

11. Method according to claim 10, **characterized by** the following steps or measures:
- attaching the second closure onto the fluid connection (16) of the non-gamma-sterilizable separation unit(s);
- uncovering the passage opening (30) if the passage opening (30) is closed;
- packing the non-gamma-sterilizable separation unit(s) into the envelope;
- sterilizing the packed separation unit(s) with superheated steam with the passage opening (30) uncovered;
- after sterilizing with superheated steam: closing the passage opening (30) in the closed envelope;
- unpacking the superheated steam-sterilized separation unit(s) from the closed envelope; and
- removing the second closure immediately prior to mounting the superheated steam-sterilized separation unit(s).

12. Method according to any one of the preceding claims, **characterized in that** the medium-contacting area of the gamma-sterilizable component and/or of the gamma-sterilizable separation unit(s) remains covered by a first closure, preferably by a blind cap (40), a sterile connector or a jacket, prior to being sterilized by gamma radiation and until the gamma-sterilized component and/or the gamma-sterilized separation unit(s) are mounted.

13. Method according to claim 12, **characterized in that** the gamma-sterilizable component comprises a plurality of interconnectable manifold assemblies for connecting a plurality of separation units (12) and/or at least one connecting hose line (20), wherein the gamma-sterilizable component has at least one fluid connection (16) which is covered by the first closure.

14. Method according to any one of the preceding claims, **characterized in that** the sterile barrier for the gamma-sterilizable component and/or the gamma-sterilizable separation unit(s) comprises a closed primary packaging which completely surrounds them.

15. Method according to any one of the preceding claims, **characterized in that** the superheated steam-sterilized separation unit(s) and the gamma-sterilized component and the gamma-sterilizable separation unit(s) are mounted on a safety cabinet or in a clean room, preferably in an area of a clean room that is separated by walls.

## Revendications

1. Procédé de maintien d'une pureté microbiologique d'un grand dispositif jetable (10) pour l'exécution d'un processus à technique de bioprocédé dans la fabrication commerciale avec un grand débit de milieu, dans lequel le dispositif jetable (10) comprend
au moins une composante stérilisable par rayonnement gamma, qui est formée dans un matériau approprié pour une stérilisation par rayonnement gamma, et
plusieurs unités de séparation sous la forme de capsules filtrantes, de colonnes chromatographiques ou d'adsorbeurs membranaires, dont au moins l'une contient un matériau non approprié pour une stérilisation par rayonnement gamma et est ainsi non stérilisable par rayonnement gamma,
dans lequel tant la composante que les unités de séparation présentent respectivement une région qui, lors de l'exécution du processus à technique de bioprocédé, entre en contact physique avec un milieu de processus, et
dans lequel le procédé comprend les étapes suivantes :
a) stérilisation par rayonnement gamma de la composante stérilisable par rayonnement gamma et de la/des unité(s) de séparation stérilisable(s) par rayonnement gamma;
b) protection de la région, en contact physique avec le milieu, de la composante stérilisable par rayonnement gamma et de la/des unité(s) de séparation stérilisable(s) par rayonnement gamma, respectivement par une barrière stérile;
c) stérilisation avec de la vapeur brûlante, individuellement ou en groupe, de la/des unité(s) de séparation non stérilisable(s) par rayonnement gamma en tant que sous-unité;
d) protection de la région, en contact physique avec le milieu, de la/des unité(s) de séparation non stérilisable(s) par rayonnement gamma, respectivement par une barrière stérile;
e) retrait de la barrière stérile; et
f) montage de la composante stérilisée par rayonnement gamma et de la/des unité(s) de séparation stérilisée(s) par rayonnement gamma ainsi que de la/des unité(s) de séparation stérilisée(s) par vapeur brûlante dans le dispositif jetable (10) immédiatement après le retrait de la barrière stérile,
dans lequel la au moins une composante stérilisable par rayonnement gamma comprend au moins un tube de liaison (18) rigide avec des raccordements fluidiques contre lesquels sont raccordés, après assemblage du dispositif jetable (10), plusieurs unités de séparation.

2. Procédé selon la revendication 1, **caractérisé en ce que** le laps de temps entre le retrait d'une barrière stérile et le montage de la composante stérilisée par rayonnement gamma correspondante ou de la/des unité(s) de séparation stérilisée(s) par rayonnement gamma, ou de la/des unité(s) de séparation stérilisée(s) par vapeur brûlante correspondante(s) dure respectivement moins de quatre minutes, de préférence moins de deux minutes, davantage de préférence moins d'une minute et encore davantage de préférence moins d'une demi-minute.

3. Procédé selon la revendication 1, **caractérisé en ce que** l'étape de la protection de la région, en contact physique avec le milieu, de la composante stérilisable par rayonnement gamma et de la/des unité(s) de séparation stérilisable(s) par rayonnement gamma et/ou de la/des unité(s) de séparation non stérilisable(s) par rayonnement gamma, respectivement par une barrière stérile, comprend un recouvrement complémentaire de la région, en contact physique avec le milieu, de la composante stérilisable par rayonnement gamma ou de la/des unité(s) de séparation stérilisable(s) par rayonnement gamma avec au moins une première fermeture, et/ou un recouvrement complémentaire de la région, en contact physique avec le milieu, de la/des unité(s) de séparation non stérilisable(s) par rayonnement gamma avec au moins une deuxième fermeture,
l'étape du retrait de la barrière stérile comprend un retrait ultérieur de la première et/ou de la deuxième fermeture, et
l'étape du montage de la composante stérilisée par rayonnement gamma ou de la/des unité(s) de séparation stérilisée(s) par rayonnement gamma, ou de la/des unité (s) de séparation stérilisée(s) par vapeur brûlante dans le dispositif jetable (10) survient immédiatement après le retrait de la première ou de la deuxième fermeture.

4. Procédé selon la revendication 3, **caractérisé en ce que**
le laps de temps entre le retrait de la barrière stérile respective et le montage de la composante stérilisée par rayonnement gamma correspondante, dotée d'une première fermeture, ou de la/des unité(s) de séparation stérilisée(s) par rayonnement gamma, ou de la/des unité(s) de séparation stérilisée(s) par vapeur brûlante correspondante (s), dotée(s) **d'une** deuxième fermeture, dure respectivement moins de trois heures, de préférence moins de deux heures, davantage de préférence moins d'une heure et encore davantage de préférence moins d'une demi-heure, et
le laps de temps entre le retrait de la première ou de la deuxième fermeture et le montage de la composante stérilisée par rayonnement gamma correspondante ou de la/des unité(s) de séparation stérilisée(s) par rayonnement gamma, ou de la/des unité(s) de séparation stérilisée(s) par vapeur brûlante dure respectivement moins de deux minutes, de préférence moins d'une minute et davantage de préférence moins d'une demi-minute.

5. Procédé selon la revendication 3 ou 4, **caractérisé en ce que** la première et/ou la deuxième fermeture forme une barrière réduisant la pénétration de particules avec une mesure de fente d'au plus 100 µm, de préférence d'au plus 50 µm, davantage de préférence d'au plus 10 µm.

6. Procédé selon l'une des revendications précédentes, **caractérisé en ce que** l'étape b) survient avant l'étape a) et/ou l'étape d) survient avant l'étape c).

7. Procédé selon l'une des revendications précédentes, **caractérisé en ce que** pour la/les unité(s) de séparation stérilisée(s) par vapeur brûlante, la barrière stérile comprend une enveloppe fermée entourant complètement la/les unité(s) de séparation stérilisée(s) par vapeur brûlante.

8. Procédé selon la revendication 7, **caractérisé en ce que** l'enveloppe est conçue dans une toile non tissée imperméable à l'eau mais perméable à la vapeur d'eau.

9. Procédé selon l'une des revendications précédentes, **caractérisé en ce que** la région, en contact physique avec le milieu, de la/des unité(s) de séparation non stérilisable(s) par rayonnement gamma est recouverte par une deuxième fermeture, en particulier un couvercle (26), avant la stérilisation avec de la vapeur brûlante et jusqu'au montage de la/des unité(s) de séparation stérilisée(s) par vapeur brûlante, dans lequel la deuxième fermeture présente au moins une ouverture de passage (30) qui peut être libérée et fermée de façon sélective.

10. Procédé selon l'une des revendications précédentes, **caractérisé en ce que** la au moins une unité de séparation non stérilisable par rayonnement gamma présente un raccordement fluidique (16) qui est recouvert par une deuxième fermeture.

11. Procédé selon la revendication 10, **caractérisé par** les étapes ou mesures suivantes :
- amenée de la deuxième fermeture sur le raccordement fluidique (16) de la/des unité(s) de séparation non stérilisable(s) par rayonnement gamma;
- libération de l'ouverture de passage (30) dans le cas où l'ouverture de passage (30) est fermée;
- empaquetage dans l'enveloppe de la/des unité(s) de séparation non stérilisable(s) par rayonnement gamma;
- stérilisation avec de la vapeur brûlante de la/des unité(s) de séparation stérilisée(s) empaquetée(s) lorsque l'ouverture de passage (30) est libérée;
- après la stérilisation avec de la vapeur brûlante : fermeture de l'ouverture de passage (30) dans l'enveloppe fermée;
- dépaquetage de la/des unité(s) de séparation stérilisée(s) par vapeur brûlante hors de l'enveloppe fermée; et
- enlèvement de la deuxième fermeture immédiatement avant le montage de la/des unité(s) de séparation stérilisée(s) par vapeur brûlante.

12. Procédé selon l'une des revendications précédentes, **caractérisé en ce que** la région, en contact physique avec le milieu, de la composante stérilisable par rayonnement gamma ou de la/des unité(s) de séparation stérilisable(s) par rayonnement gamma demeure recouverte par une première fermeture, de préférence par un capuchon aveugle (40), un raccord stérile ou une gaine, avant la stérilisation par rayonnement gamma et jusqu'au montage de la composante stérilisée par rayonnement gamma ou de la/des unité(s) de séparation stérilisée(s) par rayonnement gamma.

13. Procédé selon la revendication 12, **caractérisé en ce que** la composante stérilisable par rayonnement gamma comprend une pluralité de modules de distributeur pouvant être reliés entre eux pour relier plusieurs unités de séparation (12) et/ou au moins une conduite souple de raccordement (20), dans lequel la composante stérilisable par rayonnement gamma présente au moins un raccordement fluidique (16) qui est recouvert par la première fermeture.

14. Procédé selon l'une des revendications précédentes, **caractérisé en ce que** pour la composante stérilisable par rayonnement gamma ou la/les unité(s) de séparation stérilisable(s) par rayonnement gamma, la barrière stérile comprend un conditionnement fermé entourant complètement celles-ci.

15. Procédé selon l'une des revendications précédentes, **caractérisé en ce que** la/les unité(s) de séparation stérilisée(s) par vapeur brûlante ainsi que la composante stérilisable par rayonnement gamma et la/les unité(s) de séparation stérilisable(s) par rayonnement gamma sont montées sur un établi de sécurité ou dans une salle blanche, de préférence dans une région séparée par des parois d'une salle blanche.
